# EUROPEAN PATENT APPLICATION

(11) **EP 3 420 911 A1**
(43) Date of publication of application: **02.01.2019**
(21) Application number: 18174980.5
(22) Date of filing: 30.05.2018
(51) Int. Cl.: A61B 6/12, A61B 6/00, A61B 6/03

(54) **METHOD FOR ACQUIRING X-RAY DATA, X-RAY DEVICE, COMPUTER PROGRAM AND ELECTRONICALLY READABLE STORAGE MEDIUM**

(30) Priority: 27.06.2017 EP 17178141
(71) Applicant: Siemens Healthcare GmbH, 91052 Erlangen (DE)
(72) Inventor: Meyer, Andreas, 91088 Bubenreuth (DE)

(57) **Abstract**

Method for acquiring at least a part of an x-ray data set with at least one x-ray device (1), the x-ray data set comprising at least one first x-ray image data set and at least one second x-ray image data set acquired separated in time, which are to be evaluated together, wherein both x-ray image data sets show a region of interest of a patient (6) which is subject to heart motion at different phases of a heart cycle (21), whereby the second x-ray image data set is acquired triggered at a beginning of each heart cycle (21) covered by the acquisition and with an acquisition rate such that the phases of the acquired x-ray image data set match phases of the first x-ray image data set.

## Description

The invention concerns a method for acquiring at least a part of an x-ray data set with at least one x-ray device, the x-ray data set comprising at least one first x-ray image data set and at least one second x-ray image data set acquired separated in time, which are to be evaluated together, wherein both x-ray image data sets show a region of interest of a patient, which is subject to heart motion, at different phases of a heart cycle. The invention also concerns an x-ray device, a computer program and an electronically readable storage medium.

Heart diseases close to the human heart, for example coronary artery chronic total occlusion (CTO), are one of the leading causes of death of patients. Thus, minimally invasive operation techniques have been developed to treat patients suffering from heart diseases. For example, to treat CTO, an available procedure is percutaneous coronary intervention (PCI), which relies on the insertion of a catheter through the obstructed artery to release the occlusion.

In general, instruments, for example catheters or guide wires, are introduced into the patient and guided to the heart structures, for example coronary blood vessels. During this kind of intervention, the practitioner has to be able to track the progress of the instruments in the blood vessels. As these instruments are usually well visible in x-ray images, in particular fluoroscopy images, navigation in the blood vessel system is usually performed using fluoroscopy imaging. However, the anatomical structures of interest are more or less transparent to x-rays, meaning they are not visible in fluoroscopy images without using contrast agents. Contrast agents, however, empty from the region of interest very quickly due to blood flow, such that the following clinical workflow was proposed:
1. Acquire a contrast-enhanced first series of x-ray images,
2. Display a contrast-enhanced reference image from this series in parallel to a current fluoroscopy image using the same acquisition geometry, in particular the same projection direction,
3. Side-by-side guidance of the interventional instrument, tracking its motion in comparison to the displayed static contrast-enhanced reference image,
4. Recurring, short contrast agent injections to ensure using the correct path for the instrument in the vessel tree or the coronary chamber.

New approaches segment interesting anatomical structures, in particular heart structures, from previous contrast-enhanced acquisitions and view these segmentation results with current x-ray images, in particular fluoroscopy images. In another known embodiment, pre-operational image data, for example magnetic resonance data (MR data) or computer tomography data (CT data) are used for segmentation and overlay. These methods aim at reducing the amount of contrast agent during the procedure and providing a faster, safer and more precise intervention.

Fusion techniques and overlay techniques are already well-known in the case of anatomical structures showing only little movement, for example in interventional neurological radiology. In this field, two dimensional or three dimensional road map techniques are used to provide anatomical information and position information of the instrument in one fused or overlaid image during the interventional procedure.

However, known static fusion and overlay techniques do not allow precise fusion and/or overlay of interesting structures regarding the heart region of the patient, since this region is subject to heart motion and breathing motion. Thus, these motions have to be compensated when fusing and/or overlaying x-ray images of the anatomy and the instrument, or, in general, evaluating image data sets acquired separated in time together. In the case of interventional guidance during an operation, in particular a minimally invasive intervention, these movement compensating techniques are called dynamic road mapping techniques.

In US 2015/0087972 A1, dynamic overlay of anatomy modeled from multi-cycle (21) angiography to fluoroscopy, that is, an overlay that changes over time with the heart and/or breathing cycles (21) of anatomical structures on fluoroscopy X-ray images, is provided. The dynamic overlay of the anatomy is based on fusing candidate overlays from a multi-cycle (21) angiography sequence and prediction. Candidate overlays of these anatomical structures on the fluoroscopy images are created based on angiographic anatomy from different cycles (21). By tracking the medical devices and anatomical structures during only a few heart cycles (21) in an angiography sequence, a model for their motion during the intervention is created. The position of anatomy across several frames in fluoroscopy imaging is predicted using the model for motion. By using the prediction model and the candidate overlays, the dynamic overlay of the anatomical structures on the fluoroscopy images is provided to assist the practitioners during surgical interventions.

It has been shown that methods like these have certain disadvantages. In particular, when overlaying x-ray images, inaccuracies occur, since, among others, the motion of the blood vessel is too fast, so that usual acquisition rates of for example 15 images per second do not suffice to sample the heart motion adequately. A possible solution would be to increase the acquisition rate to obtain a sufficient sample frequency for the heart motion. This approach, however, is diametrically opposed to the trend of decreasing patient x-ray dose or to keep patient dose low, respectively.

It is an object of the current invention to provide a method for more robustly and accurately evaluating x-ray images associated with heart cycle phases together.

This object is achieved by providing a method according to claim 1, an x-ray device according to claim 13, a computer program according to claim 14 and an electronically readable storage medium according to claim 15.

In a method as initially described, the invention proposes to acquire at least the second x-ray image data set triggered at a beginning of each heart cycle covered by the acquisition and with an acquisition rate such that the phases of the acquired x-ray image data set match phases of the first x-ray image data set.

The first image data set comprises images at equidistant phases of the heart cycle, wherein in particular one imaged phase coincides with a beginning of the heart cycle. This can be achieved by also acquiring it triggered, however, it can also be realized by using corresponding reconstruction parameters during a reconstruction process. Having such a first image data set, the second x-ray image data set may be acquired triggered at the beginning of each heart cycle included in the acquisition of the second image data set, and with an acquisition rate such that the phases of the second image data set at least essentially match phases in each of the heart cycles covered by the first image data set.

It is noted at this point that it is general knowledge that, usually, the heart rate, in particular the length of a heart cycle, usually does not change abruptly and remains essentially constant at least at the time scales used for imaging. That is, while the following heart cycle and its duration is, of course, not known in advance, it can be predicted with a very high probability as being like the last few heart cycles. This predictability allows choosing the acquisition rate such that the phases of the first and second image data sets at least essentially match. In other words, the acquisition rate is chosen on the predicted heart cycles based on information on past heart cycles, in particular based on the assumption of the heart rate remaining at least essentially constant. Thus, "with an acquisition rate such that the phases of the acquired x-ray image data set match phases of the first x-ray image data set" is to be understood as describing the process or step of choosing the acquisition rate, not the result to be (in any case) achieved. The most common assumption used in this step will be, as explained, the heart rate remaining at least essentially constant over the heart cycles included in the acquisition. Therefore, the length of the heart cycles are known and the acquisition rate can easily be chosen such that the phases of the second image data set at least essentially match the phases of the first image data set, as long as this assumption holds true. The heart beat rate at the beginning of the acquisition of the the second image data set may be measured as known in the state of the art.

Alternatively, of course, it is possible to influence the heart motion such that the heart rate remains constant by using a cardiac pacemaker, as has also been proposed in the state of the art. However, due to the known stability of the heart rate, which remains essentially constant over a typical image acquisition, a pacemaker is not needed according to the invention. Exceptional cases, in which the heart rate does not remain constant during the triggered acquisition, will be discussed below. In particular, the acquisition rate can be adapted, and/or the acquisition can be aborted and, for example, restarted as soon the heart rate remains (at least essentially) constant again.

The x-ray source of the x-ray device is thus not controlled continuously with the acquisition rate, but synchronized with the beginning of each new heart cycle, such that x-ray images are, for each heart cycle covered by the acquisition, always recorded at the same positions in the heart cycle. In this description, the term "phase of the heart cycle" or "heart cycle phase" is used to refer to a specific moment during a heart cycle. In particular, a heart cycle phase may thus describe a certain time interval during the heart cycle, during which no strong movements occur. In particular, if a heart cycle is denoted as running from 0 % to 100 %, phases may be denoted by percentage values.

The herein described method assures that a defined starting point during each heart cycle is used, such that when using a corresponding acquisition frequency, additional x-ray images taken during these heart cycles will always show corresponding phases, in particular also in acquisitions of x-ray image data sets over multiple heart cycles within the same x-ray image data set. There can be at least three images taken during each heart cycle, however, the invention also works in principle for the acquisition of two images during a heart phase, such that the term "rate" shall also cover this case. The electrocardiogram (ECG) triggering as defined above need not be restricted to the second x-ray image data set, but is preferably also used for the first x-ray image data set, while in particular using the same trigger event in the heart cycle, which is preferably the R-wave. In particular in acquisitions of the first x-ray image data set spanning multiple heart cycles, it will in this manner be assured that x-ray images recorded in different heart cycles will also correspond in heart cycle phase.

The invention thus allows ECG-synchronous acquisition of x-ray data sets, increasing the accuracy and robustness in comparison to a non-ECG-synchronous acquisition technique.

As already mentioned, the triggering is preferably performed at the R-wave of the heart cycle. In this embodiment the beginning of each heart cycle covered by the acquisition which triggers the acquisition of the second x-ray image data set is the R-wave of the heart cycle. The R-wave can be detected reliably by known ECG measuring devices and has already been proposed as a trigger for certain imaging applications. Of course, besides the R-wave also other significant features of the ECG can be used to define a beginning of the heart cycle, for example the Q-, S- or T-wave.

In an especially advantageous case of application, at least one of the at least one first x-ray image data set is a contrast-enhanced anatomy data set and at least one of the at least one second image data set comprises intra-operational fluoroscopy images, wherein the fluoroscopy images are displayed overlaid or fused with anatomy images of the first x-ray image data set having the same heart cycle phase. The first x-ray image data set thus shows the anatomy and is used as a background for current fluoroscopy images showing an instrument used in a minimally invasive intervention. Such a contrast-enhanced anatomy data set can also be called "dynamic road map". Thus, the invention allows ECG-synchronous acquisition of the dynamic road map, which in turn can be ECG-synchronously overlaid to the fluoroscopy images showing the medical instrument used in the minimally-invasive intervention. Thus, the accuracy of overlaying/fusing the dynamic road map is considerably increased. It is noted that, since the phases of the fluoroscopy images correspond to the phases of the contrast-enhanced anatomy data set by adequately controlling their acquisition anyway, it is sufficient to record the dynamic road map over one heart cycle with one contrast agent filling only, while, according to the state of the art, multiple heart cycles should be used for acquisition of a dynamic road map. Thus, the invention allows to reduce patient x-ray dose and contrast agent. Of course, the invention can also be applied to other embodiments in which image data sets are evaluated together, for example when comparing structures in these data sets or deriving other results.

Preferably, the contrast-enhanced anatomy data set and the fluoroscopy images are acquired using the same x-ray device, in particular with the patient positioned for the operation to be performed. In this manner, the first x-ray image data set and the second x-ray image data set are inherently registered to one another, facilitating the combined evaluation/overlay or fusion. The x-ray device can preferably be an angiography device, in particular having a C-arm, to which the x-ray source and the x-ray detector are mounted at opposing ends.

As already mentioned, in preferred embodiments, the contrast-enhanced anatomy data set is also acquired triggered at the beginning of each heart cycle covered by the acquisition. If multiple heart cycles are covered, at the beginning of each heart cycle, synchronisation occurs by re-starting the acquisition timing with the adequate acquisition rate. Of course, also the first heart cycle or, if only one heart cycle is acquired, the only heart cycle is acquired ECG-triggered by starting the acquisition at the beginning of the heart cycle, preferably the occurrence of the R-wave. Thus, the herein described ECG-triggering is also used during the acquisition of the contrast-filled blood vessels over at least one heart cycle. The recorded x-ray images are stored as dynamic road map.

In a concrete embodiment, the contrast-enhanced anatomy data set may comprise digital subtraction angiography images (DSA images), wherein mask images and fill images are matched in heart cycle phase by acquiring them triggered at the beginning of each heart cycle covered using an acquisition rate such that the phases of the mask images and the fill images match. It is thus possible to acquire mask images during at least one heart cycle natively, that is, without using contrast agent, and acquire fill images during at least one further heart cycle after contrast agent has been administered, wherein, due to both acquisitions being performed ECG-synchronized, the phases match such that the mask images may be subtracted from the fill images to create a dynamic DSA road map. Such a dynamic DSA road map facilitates blood vessel segmentation significantly since the detection algorithms for contrasts-filled blood vessels are not distracted by other native structures in the images.

In an especially preferred embodiment, the acquisition rate is chosen according to a current heart rate or adapted to a current heart rate. A possible parametrisation for the acquisition rate may be to require a certain number, for example at least three, of x-ray images to be taken during each heart cycle, such that, depending on a current heart rate, the acquisition rate can be chosen such that the corresponding number of x-ray images equidistant in time can be acquired. Thus, the synchronization may also extend to the acquisition rate. If, in an example, 15 images should be acquired during each heart cycle, the acquisition rate can be chosen appropriately. If the heart rate is 60 beats per minute and 15 images are to be acquired, each 67 milliseconds a new x-ray image is taken. With each beginning of a new heart cycle, in particular R-wave, the timing has to be restarted/synchronized to acquire x-ray images in the same heart cycle phases.

Independent of the definition of the acquisition rate itself, that is, also if an acquisition rate, for example 15 images per second, is predetermined, it is important to remember that, as already noted, the heart rate and thus the duration of heart cycles may change during an acquisition procedure or between acquisition procedures, for example between acquisition of the first x-ray image data set and the second x-ray image data set. In particular, the inventive method can also react to changes in heart rate. Usually, ECG measuring devices also measure the heart rate anyway during an acquisition, for example by continuously averaging over a number of the last heart cycles. If the heart rate changes, the acquisition rate can be adapted to obtain matching phases of single images as accurately as possible. This adaptation may also take place dynamically during an acquisition process, for example during acquisition of the second image data set.

In a concrete embodiment, the acquisition rate is chosen as a reference acquisition rate describing the phases to be acquired during a reference heart rate multiplied with the ratio of the current heart rate to the reference heart rate. The reference heart rate can be defined by the first heart cycle acquired and/or the reference acquisition rate can be chosen by a user, as further discussed below. The timing to be used, for example defined by the first heart cycle acquired, can also, for example, be redefined in percentage values between 0 and 100 %, wherein the duration 0 to 100 % corresponds to the R-R interval. It is now possible to adapt the timing to natural fluctuations of the duration of the R-R interval/heart cycle. In the case of the creation of a dynamic road map and the subsequent application of this road map to fluoroscopy images, the acquisitions follow each other close in time such that the heart rate and thus the motion pattern of the heart should not have changed severely.

Preferably, the triggered acquisition is started only if a stability criterion describing the heart rate remaining constant is fulfilled. To exclude time intervals of a regular heartbeat, it can be provided to verify if the heart rate is stable enough to acquire x-ray images using ECG triggering as described. A stability criterion is used ensuring that the heart rate does not fluctuate in average more than a predetermined threshold. The fulfilment of the stability criterion can also be displayed to a user starting the acquisition manually.

The acquisition rate can be chosen dependent on user input. The user input can be, as already mentioned, abstract, for example choosing a certain number of images per heart cycle to be acquired. However, the user can also choose a certain acquisition rate which, of course, can be adapted later on during the acquisition as described above. It is also possible that the user input is constrained, for example, if certain requirements are to be met. If, for example, the first x-ray image data set is a four-dimensional CT data set with a predetermined number of phases for which a volume image is comprised, the user can be confined to using integer divisors of the number of phases contained in the four-dimensional CT data set. Corresponding constraints can also result when using a dynamic route map acquired before. It is noted that reducing the number of phases imaged for the second x-ray image data set can also be advantageous to further reduce patient x-ray dose.

In an embodiment, at least one of the at least one first x-ray image data set can be a four-dimensional CT data set comprising volume images for phases equidistant in time, wherein the acquisition rate for the remaining x-ray data set is chosen such that all acquired phases match a phase of a volume image of the CT data set. In the method described herein, also four-dimensional (3D+t) CT data sets can be used, which usually comprise a certain timing which can be transferred to the acquisition of further x-ray data, in particular second x-ray image data sets. For example, the duration of the heart cycle can again be assumed as 100 % such that the timing described by the phases of the volume images of the four-dimensional CT data set can be converted into the corresponding timing during acquisition of further x-ray data, for example fluoroscopy images, in particular also adapting the acquisition rate to the current heart rate.

In the application of dynamic road mapping, the four-dimensional CT data set can be considered pre-operational 3D+t data, which can also be used as showing the anatomy in a road mapping procedure, assuming that heart motion is similar, even if the heart rates differ. It is noticed that in this particular application, also first image data sets from other modalities, which can be registered to the x-ray device/x-ray data, can be used as four-dimensional pre-operational data sets, for example magnetic resonance (MR) data.

It is to be noted that often, the region of interest is not only subject to heart motion, but also subject to breathing motion. While certain first and/or second x-ray image data sets may also be acquired during breath holds, in a preferred embodiment a breathing motion correction can be added. Therefore, it can be provided that the x-ray data set is corrected for breathing motion, in particular by a pixel shift algorithm. Suitable algorithms for breathing motion correction, in particular pixel shift algorithms, are already well-known from the state of the art so that they shall not be further discussed here.

An x-ray device according to the invention comprises a control device adapted to perform a method according to the invention. All remarks relating to the inventive method are also applicable to the inventive x-ray device. The x-ray device can preferably be an angiography device, in particular having a C-arm with an x-ray source and an x-ray detector at opposing ends thereof.

In general, an x-ray device according to the invention may in particular comprise an acquisition arrangement comprising an x-ray source and an x-ray detector and/or an ECG measuring device for generating a trigger signal at the beginning of each heart cycle to be sent to the control device. The trigger signal can then be used to control the x-ray source to allow ECG-synchronized acquisition of x-ray images. In concrete embodiments, the control device may comprise a control unit controlling the acquisition of x-ray data synchronized with the trigger signal using an acquisition rate, and an image evaluation unit for assigning a heart cycle phase to acquired x-ray images and evaluating the first x-ray image data set and the second x-ray image data set together. The control device may further optionally comprise an adaptation unit for adapting the acquisition rate to a current heart rate and/or a user interface for interacting with a user and/or other units relating to other preferable embodiments of the method according to the invention.

A computer program according the invention can be directly loaded into a storage device of a control device of an x-ray device and comprises computer program means to perform the steps of a method described herein when the computer program is executed in the control device of the x-ray device. The computer program may be stored on an electronically readable storage medium according to the invention, which thus comprises electronically readable control information stored thereon, which at least comprises a computer program according to the invention. The control information is configured to perform a method described herein when using the storage medium in a control device of an x-ray device. The storage medium may be a non-transitory storage medium, for example a CD-ROM.

Additional details and advantages of the current invention can be taken from the following description of preferred embodiments in conjunction with the drawings, in which:
- Fig. 1: shows an x-ray device according to the invention,
- Fig. 2: shows a flow chart of a method according the invention,
- Fig. 3: shows an example for the applied timing.

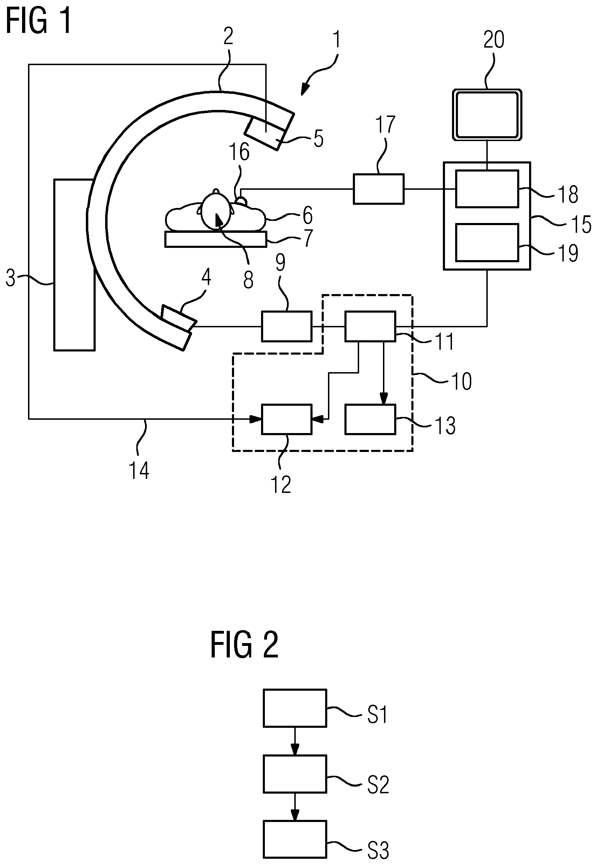

Fig. 1 shows an inventive x-ray device 1, which in this case is an angiography device having a C-arm 2 supported by a support 3. To opposite ends of the C-arm 2, an x-ray source 4 and an x-ray detector 5 are mounted. The C-arm may be positioned relatively to a patient 6 supported by a patient table 7 to realise different acquisition geometries.

The x-ray device 1 can in particular be used to survey a minimally invasive intervention/operation of a patient 6, in particular for tracking a medical instrument 8 inserted into a patient 6, for example a catheter.

A voltage for the x-ray source 4, here an x-ray tube, is provided by an x-ray generator 9 which in turn receives control signals from control device 10, which is configured to perform the steps of a method according the invention. The control device 10 in this case comprises at least a control unit 11, an image evaluation unit 12 and a user interface 13. The image evaluation unit 12 also receives raw x-ray data from the x-ray detector 5 according to arrow 14.

The x-ray device 1 further comprises an ECG measuring device 15 having electrodes 16 to be placed on the patient 6, a signal amplifier 17, an evaluation unit 18 and a trigger signal generating unit 19. The ECG measuring device 15 may also comprise display means 20. Display means 20 may, for example, indicate fulfilment of a stability criterion describing the heart rate remaining constant in a certain interval. If the stability criterion is fulfilled, an ECG-synchronized acquisition of x-ray data may be performed as further described below.

An example of a method according to the invention will now be described with respect to fig. 2. In this example, a dynamic road mapping procedure during a medical intervention is to be performed.

In a first optional step S1, a user may input data regarding the acquisition rate using user interface 13, in this embodiment by selecting a number of x-ray images to be acquired during a heart cycle. Different values may be chosen for acquiring a first x-ray image data set, here a contrast-enhanced anatomy data set (dynamic road map), and a second x-ray image data set, in this case fluoroscopy images showing the instrument 8. However, the second value should be a divisor of the first value to facilitate matching heart cycle phases, as further described below. It should also be noted that, if pre-operative image data, for example a four-dimensional CT data set, is used, which already provides a given number of volume images at certain phases during the heart cycle, this may also constrain the number of x-ray images that can be acquired during each heart cycle regarding fluoroscopy images to be overlaid onto the three-dimensional volume images of the pre-operative image data.

In a step S2, the contrast-enhanced anatomy data set is to be acquired using the x-ray device 1. In an embodiment, a contrast agent is administered and a certain number of x-ray images are recorded during a single heart cycle. If the stability criterion is fulfilled, the ECG measuring device 15 transmits an ECG trigger signal at the beginning of a heart cycle, here when detecting the R-wave, to the control device 10, in particular the control unit 11, which instantly generates a series of control signals to the x-ray generator 9 implementing an acquisition rate, in this case calculated from the duration of a heart cycle in the current heart rate and the user input named above. If the heart rate is 60 beats per minute and the number of images to be acquired during a heart cycle is 15, the corresponding acquisition rate is 15 images per second, i.e. one image every 67 milliseconds.

The result of this acquisition is stored as a dynamic road map in a storage device of the control device 10, in particular the image evaluation unit 12.

It is, however, also possible to acquire x-ray images of the contrast-enhanced anatomy data set over multiple heart cycles, wherein each time a trigger signal is received from the ECG measuring device 15, a control unit 11 re-starts the acquisition process, thus synchronizing with the heart cycle to image the same phases of the heart cycle in each of the heart cycles covered by the acquisition.

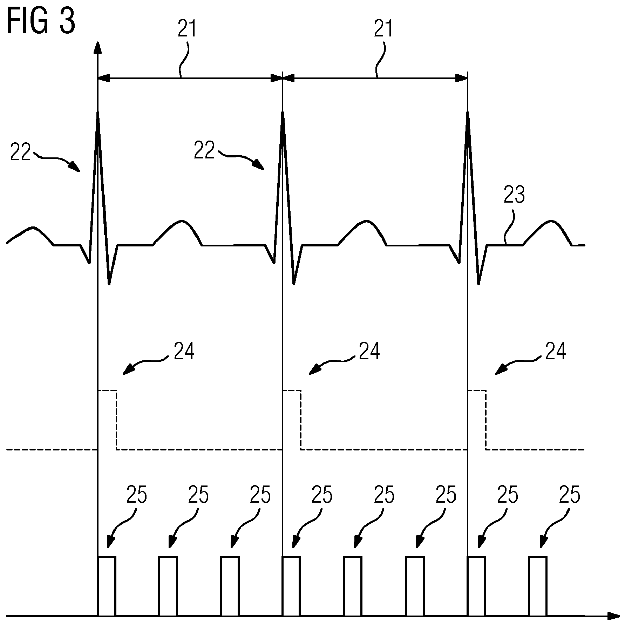

This is depicted in a simplified example of three acquisitions per heart cycle 21 in fig. 3. As can be seen, the beginning of each heart cycle 21 and thus the duration of a heart cycle 21 is defined by respective R-waves 22 in the ECG signal 23 measured by the ECG measuring device 15. As soon as an R-wave 22 is detected, a corresponding trigger signal 24 is transmitted to the control device 10, as discussed above. In response to the trigger signal 24, which acts as a synchronization signal, an acquisition timing is started in control unit 11, beginning to record x-ray images of the region of interest in the patient, which is subject to heart motion, with a corresponding acquisition rate, here, assuming a heartbeat rate average of 60 beats per minute, of three images per second and, in other words, one image every 333 seconds. The corresponding control signals 25 from the control unit 11 to the x-ray generator 9 are shown in the bottom line of fig. 3.

As can be seen, independently of the duration of a particular heart cycle 21, the imaging always starts anew on reception of a trigger signal 24, assuring acquisition of x-ray images in phases of the heart cycle as similar as possible.

In a concrete embodiment, the contrast-enhanced anatomy data set can be acquired as a DSA data set, wherein during a first heart cycle mask images are acquired without contrast agent in the region of interest of the patient. Subsequently, after a contrast agent has been administered, during a second heart cycle, fill images are acquired using the same timing as during the acquisition of the mask images in a further heart cycle. Should the heart rate have changed between these acquisitions, the acquisition rate can advantageously be adapted to the new heart rate, using the heart rate during acquisition of the mask images as a reference heart rate and the acquisition rate during the acquisition of the mask images as reference acquisition rate. In this manner, the timing is reused in an adapted form to obtain mask images and fill images similar in heart cycle phase, such that the mask images can be subtracted from the fill images corresponding in heart cycle phase to obtain DSA images.

During the intervention, in a step S3, the timing used to obtain the contrast-enhanced anatomy data set is again used to obtain fluoroscopy images of the patient 6 showing the instrument 8. Again, at the beginning of each heart cycle covered by the acquisition, when a trigger signal 24 is received, the acquisition of fluoroscopy images is started anew, thus synchronized with the heart cycles, using the acquisition rate, preferably again adapted to the current heart rate. Thus, for each heart cycle phase of a fluoroscopy image, a corresponding dynamic road map image exists in the contrast-enhanced anatomy data set such that these images corresponding in phase can be fused and/or overlaid in the evaluation unit 12. It is added at this point that the image evaluation unit 12 also assigns respective phases to acquired x-ray images according to the control signal 25/trigger signals 24.

The fused/overlaid images can then be displayed using display means 20 and/or user interface 13 to provide a navigation/tracking 8 for the user, in particular the practitioner performing the minimally invasive intervention. It has been shown that this method of ECG-synchronizing leads to a significant stabilisation of the fusion and/or overlay during dynamic road mapping procedures.

It is finally noted that the x-ray data sets acquired according to the invention and the concrete embodiments mentioned here may be subject to an additional breathing motion correction, in particular using a pixel shift algorithm.

Although the present invention has been described in detail with reference to the preferred embodiment, the present invention is not limited by the disclosed examples from which the skilled person is able to derive other variations without departing from the scope of the invention.

It is additionally noted that the person skilled in the art will, of course, exclude interpretations which illogical or which do not make technical sense. For example, it is obvious that the described method may not be meaningfully applied to dead patients or patients with severe cardiac arrhythmia.

## Claims

1. Method for acquiring at least a part of an x-ray data set with at least one x-ray device (1), the x-ray data set comprising at least one first x-ray image data set and at least one second x-ray image data set acquired separated in time, which are to be evaluated together, wherein both x-ray image data sets show a region of interest of a patient (6) which is subject to heart motion at different phases of a heart cycle (21),
**characterized in that** at least the second x-ray image data set is acquired triggered at a beginning of each heart cycle (21) covered by the acquisition and with an acquisition rate such that the phases of the acquired x-ray image data set match phases of the first x-ray image data set.

2. Method according to claim 1, **characterized in that** the triggering is performed at the R-wave (22) of the heart cycle (21) .

3. Method according to claim 1 or 2, **characterized in that** at least one of the at least one first x-ray image data sets is a contrast-enhanced anatomy data set and at least one of the at least one second image data sets comprises intra-operational fluoroscopy images, wherein the fluoroscopy images are displayed overlaid or fused with anatomy images of the first x-ray image data set having the same heart cycle (21) phase.

4. Method according to claim 3, **characterized in that** the contrast-enhanced anatomy data set and the fluoroscopy images are acquired using the same x-ray device (1), in particular with the patient (6) positioned for the operation to be performed.

5. Method according to claim 3 or 4, **characterized in that** the contrast-enhanced anatomy data set is also acquired triggered at the beginning of each heart cycle (21) covered.

6. Method according to claim 5, **characterized in that** the contrast-enhanced anatomy data set comprises digital subtraction angiography images, wherein mask images and fill images are matched in heart cycle (21) phase by acquiring them triggered at the beginning of each heart cycle (21) covered using an acquisition rate such that the phases of the mask images and the fill images match.

7. Method according to one of the preceding claims, **characterized in that** the acquisition rate is chosen according to a current heart rate or adapted to a current heart rate.

8. Method according to claim 7, **characterized in that** the acquisition rate is chosen as a reference acquisition rate describing the phases to be acquired during a reference heart rate multiplied with the ratio of the current heart rate to the reference heart rate.

9. Method according to one of the preceding claims, **characterized in that** the triggered acquisition is started only if a stability criterion describing the heart rate remaining constant is fulfilled.

10. Method according to one of the preceding claims, **characterized in that** the acquisition rate is chosen dependent on user input.

11. Method according to one of the preceding claims, **characterized in that** at least one of the at least one first x-ray image data set is a four-dimensional CT data set comprising volume images for phases equidistant in time, wherein the acquisition rate for the remaining x-ray data set is chosen such that all acquired phases match a phase of a volume image of the CT data set.

12. Method according to one of the preceding claims, **characterized in that** the x-ray data set is corrected for breathing motion, in particular by a pixel shift algorithm.

13. X-ray device (1), comprising a control device (10) adapted to perform a method according to one of the preceding claims.

14. Computer program, adapted to perform the steps of a method according to any of the claims 1 to 12 when the computer program is executed on a control device (10) of an x-ray device (1).

15. Electronically readable storage medium, on which a computer program according to claim 14 is stored.
